# EUROPEAN PATENT SPECIFICATION

(11) **EP 0 584 903 B1**
(45) Date of publication and mention of the grant of the patent: **04.10.2001**
(21) Application number: 93303576.8
(22) Date of filing: 07.05.1993
(51) Int. Cl.: C07D 417/12, C07D 209/34, C07D 413/12

(54) **Process for preparing aryl piperazinyl-heterocyclic compounds**
Verfahren zur Herstellung von Aryl-piperazinyl-heterocyclischen Derivaten
Procédé de préparation de dérivés aryl pipérazinyl-hétérocycliques

(30) Priority: 26.08.1992 US 936179
(43) Date of publication of application: 02.03.1994
(60) Divisional application: 00201940.4
(73) Proprietor: PFIZER INC., New York, N.Y. 10017 (US)
(72) Inventor: Busch, Frank R., Gales Ferry, CT 06335 (US); Allen, Douglas, New London, CT 06320 (US); Bowles, Paul, Groton, CT 06340 (US); Diroma, Sabeto A., Uncasville, CT 06382 (US); Godek, Dennis M., Hartford County, CT 06033 (US)
(74) Representative: Wood, David John

(56) References cited:
- EP-A- 0 281 309
- US-A- 5 206 366

## Description

### Background of the Invention

The present invention is directed to a novel process for preparing arylpiperazinyl-ethyl(or butyl)-heterocyclic compounds and their pharmaceutically acceptable acid addition salts.

U.S. Patent No. 4,831,031 indicates that arylpiperazinyl-ethyl(or butyl)-heterocyclic compounds may be prepared by reacting an N-arylpiperazine with a fused bicyclic compound. This coupling reaction is generally conducted in a polar solvent (such as a lower alcohol, dimethylformamide or methylisobutylketone) and In the presence of a weak base, and preferably the reaction is in the further presence of a catalytic amount of sodium iodide and a neutralizing agent for hydrochloride such as sodium carbonate.

Yevich et al., J. Med. Chem., 29, No. 3, pp. 359-369 (1986), relates to a method of producing 1-(1,2-benzisothiazol-3-yl)- and (1,2-benzisoxazol-3-yl)piperazine derivatives. Several reaction schemes are disclosed, including reaction schemes wherein coupling occurs in a free base.

All of the documents cited herein, including the foregoing, are hereby incorporated in this application in their entireties.

The present invention relates to a new and useful process for effecting coupling reactions of piperazine derivatives with alkyl halide derivatives that provide aryl piperazinyl-ethyl (or butyl)-heterocyclic compounds in greater yields than known methods. In the present invention, the coupling reaction is conducted in water. This aqueous based coupling process is not only more efficient but also has a much lower environmental burden since the handling and disposal of organic solvents are eliminated. This process has not shown formation of byproducts and does not require special isolation procedures, e.g., extractions, distillations and recrystallizations.

### Summary of the Invention

The present invention relates to a novel process of preparing compounds of the formula 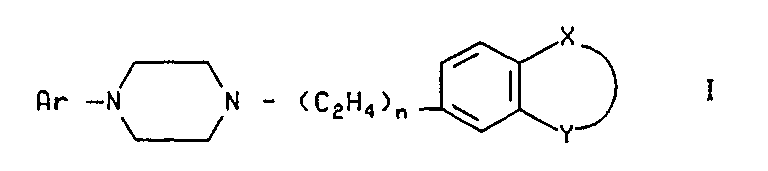 and the pharmaceutically acceptable acid addition salts thereof, wherein Ar is naphthyl optionally substituted with one to four substituents independently selected from fluoro, chloro, trifluoromethyl, methoxy, cyano and nitro; quinolyl; isoquinolyl; quinazolyl; 6-hydroxy-8-quinolyl; benzoisothiazolyl and an oxide or dioxide thereof, each optionally substituted with one or more substituents independently selected from fluoro, chloro, trifluoromethyl, methoxy, cyano, and nitro; benzothiazolyl; benzothiadiazolyl; benzotriazolyl; benzoxazolyl; benzoxazolonyl; indolyl; indanyl optionally substituted by one or two fluoro; 3-indazolyl optionally substituted by 1-trifluoromethylphenyl; and phthalazinyl;
n is 1 or 2; and
X and Y, together with the phenyl to which they are attached, form a ring system selected from quinolyl; 2-hydroxyquinolyl; benzothiazolyl; 2-aminobenzothiazolyl; benzoisothiazolyl; indazolyl; 3-hydroxyindazolyl; indolyl; spiro[cyclopentane-1,3'-indolinyl]; and oxindolyl; wherein said ring system may optionally be substituted with one to three substituents independently selected from (C₁-C₃) alkyl, or with one substituent selected from chloro, fluoro, benzoxazolyl, 2-aminobenzoxazolyl, benzoxazolonyl, 2-aminobenzoxazolinyl, benzothiazolonyl, benzoimidazolonyl, benzotriazolyl, and phenyl optionally substituted by one chloro or fluoro;
which comprises reacting a piperazine of the formula 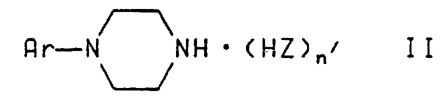 wherein Z is fluoro, chloro, bromo, iodo, methanesulfonate, trifluoromethanesulfonate, or trifluoroacetate; n' is 0 or 1; and Ar is as defined above, with an alkyl halide containing compound of the formula 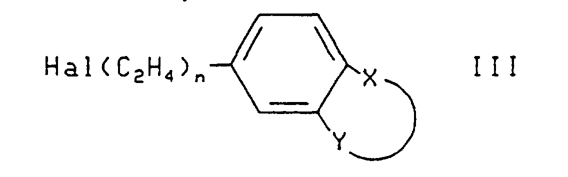 wherein n, X and Y are as defined above and Hal is fluoro, chloro, bromo or iodo, in water with a reagent to neutralize the hydrohalic acid, heating the mixture under conditions which are suitable to effect the coupling of said piperazine with said alkyl halide containing compounds, and, if desired, preparing the corresponding pharmaceutically acceptable acid addition salt. Preferably, an excess of reagent is utilized to neutralize the hydrohalic acid, and the mixture is heated to about the reflux temperature. Especially preferably, the compound of formula I is reacted with aqueous hydrochloric add to form a hydrochloride monohydrate.

The optional substitution in the naphthyl and oxindolyl may be in either ring of the naphthyl and oxindolyl group, respectively. Examples of such substitutions are 6-fluoronaphthyl, 4-methoxynaphthyl, 1-ethyloxindolyl and 6-fluorooxindolyl. The optional substitution in the indanyl is in the saturated ring of the indanyl group. Preferred substitution of the oxindolyl by (C₁-C₃)alkyl is by one to three methylene groups, or one ethyl.

Preferred compounds for use in the process of the present invention are those wherein n is 1, those wherein X and Y together with the phenyl to which they are attached form oxindolyl, those wherein Ar is naphthyl or benzoisothiazolyl, and those wherein n' is 1.

A specific preferred compound which may be prepared in accordance with the present invention is 5-(2-(4-(1,2-benzisothiazol-3-yl)-1-piperazinyl)ethyl)-6-chloro-1,3-dihydro-2H-indol-2-one hydrochloride monohydrate.

### Detailed Description of the Invention

Generally, the process of the present invention is effected in the presence of a neutralizing agent, for example, a base and including but not limited to alkali or alkaline earth metal carbonates such as sodium carbonate or potassium carbonate; bicarbonates such as sodium bicarbonate; hydrides and tertiary amines such as triethylamine or diisopropylethylamine.

Both the piperazine and the alkyl halide containing compounds are referred to herein as substrates. For the purposes of the present invention, the substrates can be present in equal molar amounts or one substrate can be present in excess.

In a preferred embodiment, the process of this invention involves the use of from about one to five molar equivalents of a neutralizing agent based on the substrate not present in excess with from about three to ten volumes of water based on the weight, e.g. grams, of the substrate not present in excess.

In a more preferred embodiment, the process of this invention involves the use of about two to three molar equivalents of a neutralizing agent based on the substrate with about five volumes of water based on the weight, e.g. grams, of the substrate which is not present in excess. In a further preferred embodiment, the neutralizing agent Is sodium carbonate.

The piperazine derivative and alkyl halide derivative are combined and heated for a time sufficient to allow the reaction to proceed, generally at least about 8 to 12 hours, and preferably for at least 10 to 12 hours. The reaction is generally conducted at a temperature of from about 80-100°C, and preferably at the reflux temperature of the reaction mixture including solvent. The reflux temperature will generally be about 100°C. The flask is cooled generally to about room temperature (20-25°C) or below but not to freezing and the product is filtered off. This reaction has not shown formation of byproducts.

The pharmaceutically acceptable acid addition salts of the compounds of formula I are prepared in a conventional manner by treating a solution or suspension of the free base (I) with about one chemical equivalent of a pharmaceutically acceptable acid. Conventional concentration and recrystallization techniques are employed In isolating the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroiodic, sulfamic, sulfonic, such as methanesulfonic, benzenesulfonic, and related acids.

The monohydrate may be prepared by reacting anhydrous 5-(2-(4-(1,2-benzisothiazol-3-yl)-1-piperazinyl)ethyl)-6-chloro-1,3-dihydro-2H-indol-2-one with aqueous hydrochloric acid. In general, this reaction takes place at temperatures of from about room temperature to about 100°C, usually from about 60° to about 65°C. Depending on the reaction temperature and other conditions, the reaction time generally ranges from about 2 hours to about 48 hours, conveniently about 3 to 24 hours. The concentration of the hydrochloric acid in the reaction solution ranges from about 0.3 to about 3.0 M, and preferably about 0.7 M.

The neuroleptic activity of the compounds prepared by the process of this invention makes them useful for treating psychotic disorders in human subjects. For example, these compounds are useful for treating psychotic disorders of the schizophrenic types, and in particular the compounds are useful for removing or ameliorating such symptoms as anxiety, agitation, excessive aggression, tension, and social or emotional withdrawal in psychotic patients.

The neuroleptic compounds of formula I and their pharmaceutically acceptable salts (hereafter also referred to as the "active compounds"), can be administered to a human subject either alone, or, preferably, in combination with pharmaceutically-acceptable carriers or diluents, in a pharmaceutical composition, according to standard pharmaceutical practice. A compound can be administered orally or parenterally. Parenteral administration includes especially intravenous and intramuscular administration. Additionally, in a pharmaceutical composition comprising an active compound the weight ratio of active ingredient to carrier will normally be in the range from 1:6 to 2:1, and preferably 1:4 to 1:1. However, in any given case, the ratio chosen will depend on such factors as the solubility of the active component, the dosage contemplated and the precise route of administration.

For oral use of an active compound the compound can be administered, for example, in the form of tablets or capsules, or as an aqueous solution or suspension. In the case of tablets for oral use, carriers which can be used include lactose and com starch, and lubricating agents, such as magnesium stearate, can be added. For oral administration in capsule form, useful diluents are lactose and dried corn starch. When aqueous suspensions are required for oral use, the active ingredient can be combined with emulsifying and suspending agents. If desired, certain sweetening and/or flavoring agents can be added. For intramuscular and intravenous use, sterile solutions of the active ingredient can be prepared, and the pH of the solutions should be suitably adjusted and buffered. For intravenous use, the total concentration of solutes should be controlled to render the preparation isotonic.

When an active compound is to be used in a human subject to treat a psychotic disorder, the daily dosage will normally be determined by the prescribing physician. Moreover, the dosage will vary according to the age, weight and response of the individual patient as well as the severity of the patient's symptoms. However, in most instances, an effective amount for treating a psychotic disorder will be a daily dosage in the range from 5 to 500 mg, and preferably 50 to 200 mg, and optionally 50 to 100 mg, in single or divided doses, orally or parenterally. In some instances it may be necessary to use dosages outside these limits.

The following examples are provided solely for the purpose of further illustration.

### Example 1

### 5-(2-(4-(1,2-benzisothiazol-3-yl)-1-piperazinyl)ethyl)-6-chloro-1,3-dihydro-2H-indol-2-one hydrochloride

In a clean 12 liter 3 necked round bottom flask there were placed 500 grams (2.28 moles) of 3-piperazinyl-1,2-benzisothiazole; 525 grams (2.28 moles) of 2-chloroethyl-6-chlorooxindole; 535 grams (5.05 moles) of sodium carbonate and 2.54 liters of water.

The materials were combined and refluxed at 100°Centigrade overnight i.e., for at least about 9 to 12 hours. (Due to the fact that this mixture bubbles up after 4 hours at reflux, adequate head space is required.)

After approximately 16 hours the flask at reflux was cooled to room temperature i.e., generally from about 20-25°C and the mixture was stirred for approximately one hour and then filtered.

863 grams of a tan colored free base was recovered (91% weight yield). High pressure liquid chromatography (HPLC) shows this product to be 94.5% pure without purification. This free base product was characterized by proton NMR, thin layer chromatography, and melting point, i.e., m.p. 218-220°C.

The free base was then converted to the hydrochloride salt and isolated in 86% weight yield and was characterized by proton NMR, thin layer chromatography, low resolution mass spectroscopy, high pressure liquid chromatography and melting point, i.e., m.p. > 300°C; MS (%): 488(1), 256(4), 232(100), 177(15); Analysis for C₂₁H₂₁ClN₄•OS•HCl•H₂O: C 52.50, H 4.71, N 11.39. Found C 52.83, H 4.93, N 11.42. This product was 99.5% pure compared to the analytical standard.

### Example 2

### Preparation of 5-[2-[4-(1,2-benzisothiazol-3-yl)-1-piperazinyl]ethyl]-6-chloro-1,3-dihydro-2H-indol-2-one

A 20-gallon glass lined tank, under a nitrogen atmosphere, was charged with 33.5 liters of water and 9.4 kilograms (kg) of sodium carbonate (dense, 89.1 moles, 3.4 eq.). The resulting mixture was stirred to give a solution. To the solution 6.4 kg of 2-chloroethyl-6-chlorooxindole (27.8 moles, 1.06 eq.) was charged, followed by 6.7 kg of 3-piperazinyl-1,2-benzisothiazole hydrochloride (26.2 moles, 1.0 eq.). This was stirred and heated to reflux (100°C). After 11 hours the reaction was sampled for high pressure liquid chromatography (HPLC) assay. The reflux was continued for another 2 hours then the reaction was cooled to 25°C and the slurry stirred for 1 hour. The product was observed and found to be essentially free from lumps and gummy matter. The product was collected by filtration on a 30" Lapp. A 14 liter water wash was added to the tank and cooled to 12°C and then used to wash the product. The cake was pulled as dry as possible, and the product was returned to the tank along with 40 liters of isopropyl alcohol (IPO). This was cooled and then stirred for 2 hours and the product was collected by filtration. The cake was washed with 13.4 liters of fresh IPO, then dried under vacuum at 30 to 40°C.

After drying, 17.3 kg of the title compound was obtained. This was in excess of the theoretical weight yield due to some residual carbonate in the crude product

### Example 3

### Recrystallization of 5-[2-[4-(1,2-benzisothiazol-3-yl)-1-piperazinyl]ethyl]-6-chloro-1,3-dihydro-2H-indol-2-one

To a clean and dry 100-gallon glass lined tank was charged 9.0 kg of the material of Example 2 and 86 gallons of tetrahydrofuran (THF). The slurry was heated to reflux and held for 1 hour. The hazy solution was then filtered through a 14" sparkler precoated with filter aid and backed with a Fulflo filter to a clean, dry, and "spec free" glass-lined tank on a lower level. The batch was concentrated by vacuum distillation. Another 8.3 kg of the material of Example 2 was dissolved in 83 gallons of THF in the upper tank. This was filtered to the lower tank. The tank lines and sparkler were rinsed with 10 gallons of THF. The batch was concentrated to about 22 gallons, then cooled to 5°C and stirred for 1 hour. The product was collected under spec free conditions by filtration on a 30". Lapp. Then 20 gallons of fresh spec free IPO were cooled in the tank and used to rinse the product cake. The product was collected and dried under vacuum at 45°C; yielding 9.05 kg of product (83.8% yield for the coupling and recrystallization).

The product matched the spectra of a standard NMR and showed the correct retention time by HPLC with 99.7% assay.

### Example 4

### Preparation of 5-[2-[4-(1,2-benzisothiazol-3-yl)-1-piperazinyl]ethyl]-6-chloro-1,3-dihydro-2H-indol-2-one

A 250 milliliter (ml) flask was charged with 25 ml of water and 6.91 grams (gm) of Na₂CO₃ (65.3 mmole, 3.4 eq.). The mixture was then stirred to give a solution. To the solution was charged 4.68 gm of 2-chloroethyl-6-chlorooxindole (20.35 mmole, 1.06 eq.) and 4.90 gm of 3-piperazinyl-1,2-benzisothiazole hydrochloride (19.2 mmole, 1 eq.). This was stirred and heated to reflux (approximately 100°C). The resulting product did not become gummy or mass together. After 14 hours, the reaction was sampled for HPLC assay. The reflux was continued for another 2 hours than the reaction was cooled to about 20°C and the slurry stirred for about 1 hour. The product was collected by filtration. The cake was pulled as dry as possible and the product was returned to the flask together with 25 ml isopropyl alcohol (IPO). The product was collected by filtration, washed with a small amount of IPO and dried under vacuum.

After drying, 7.29 gm of the title compound was obtained, representing a weight yield of 92.1%. The product matched the spectra of a standard nuclear magnetic resonance (NMR) and showed the correct retention time by HPLC with 98.6% assay.

## Claims

1. A process for preparing a compound of the formula: 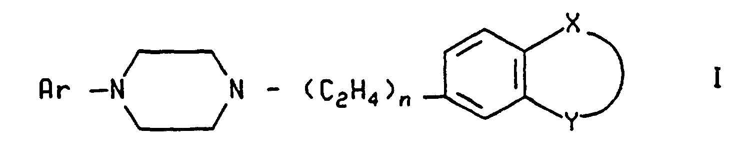 or a pharmaceutically acceptable acid addition salt thereof, wherein
Ar Is naphthyl optionally substituted with one to four substituents independently selected from fluoro, chloro, trifluoromethyl, methoxy, cyano and nitro; quinolyl; 6-hydroxy-8-quinolyl; isoquinolyl; quinazolyl; benzoisothiazolyl and an oxide or dioxide thereof, each optionally substituted with one or more substituents independently selected from fluoro, chloro, trifluoromethyl, methoxy, cyano, and nitro; benzothiazolyl; benzothiadiazolyl; benzotriazolyl; benzoxazolyl; benzoxazolonyl; indolyl; indanyl optionally substituted by one or two fluoro; 3-indazolyl optionally substituted by 1-trifluoromethylphenyl; and phthalazinyl;
n is 1 or 2; and
X and Y, together with the phenyl to which they are attached, form a ring system selected from quinolyl; 2-hydroxyquinolyl; benzothiazolyl; 2-aminobenzothiazolyl; benzoisothiazolyl; indazolyl; 2-hydroxyindazolyl; indolyl; spiro[cyclopentane-1,3'-indolinyl]; and oxindolyl; wherein said ring system may optionally be substituted with one to three substituents independently selected from (C₁-C₃)alkyl; or with one substituent selected from chloro, fluoro, benzoxazolyl, 2-aminobenzoxazolyl, benzoxazolonyl, 2-aminobenzoxazolinyl, benzothiazolonyl, benzoimidazolonyl, benzotriazolyl, and phenyl optionally substituted by one chloro or fluoro;
which comprises reacting a piperazine of the formula 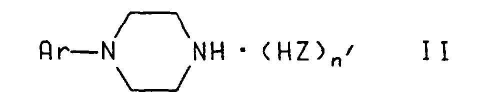 wherein Z is fluoro, chloro, bromo, iodo, methanesulfonate, trifluoromethanesulfonate, or trifluoroacetate;
n' is 0 or 1; and
Ar is as defined above, with an alkyl halide containing compound of the formula 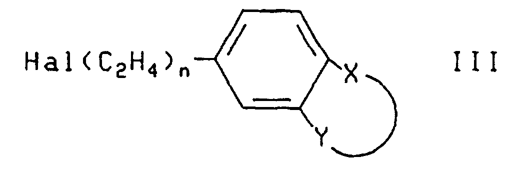 wherein n, X and Y are as defined above and Hal is fluoro, chloro, bromo or iodo, in water with a reagent to neutralize the hydrohalic acid, heating the mixture under conditions which are suitable to effect the coupling of said piperazine with said alkyl halide containing compound and, if desired, preparing the corresponding pharmaceutically acceptable acid addition salt.

2. A process according to claim 1 wherein n' = 1 and the piperazine salt is a hydrochloride or triflate salt.

3. A process according to claim 1 wherein the coupling of the piperazine with the alkyl halide containing compound is effected in the presence of a neutralizing agent.

4. A process according to claim 3 wherein from about one to five molar equivalents of a neutralizing agent based on the substrate not present in excess is used with from about three to ten volumes of water based on the weight of the substrate not present in excess.

5. A process according to claim 4 wherein about three molar equivalents of a neutralizing agent and about five volumes of water are used.

6. A process according to claim 5 wherein the neutralizing agent is selected from the group consisting of alkali metal carbonates, alkaline earth metal carbonates, bicarbonates, hydrides and tertiary amines.

7. A process according to claim 6 wherein the neutralizing agent is sodium carbonate.

8. A process according to claim 7 wherein the piperazine, alkyl halide containing compound, sodium carbonate, and water are combined and heated to reflux.

9. A process according to claim 1 wherein the mixture is heated to about reflux temperature.

10. A process according to claim 9 wherein the temperature of reflux is about 100°C.

11. A process according to claim 1 wherein the compound of the formula I is 5-(2-(4-(1,2-benzisothiazol-3-yl)-1-piperazinyl)ethyl)-6-chloro-1,3-dihydro-2H-indol-2-one.

12. A process according to claim 11, comprising the step of reacting the compound of formula I with aqueous hydrochloric add to form 5-(2-(4-(1,2-benzisothiazol-3-yl)-1-piperazinyl)ethyl)-6-chloro-1,3-dihydro-2H-indol-2-one hydrochloride monohydrate.

13. A process according to claim 1 which comprises reacting the piperazine with the alkyl halide containing compound in water and a neutralizing agent; refluxing the mixture for at least about 8 to 16 hours; cooling the mixture and filtering off the product.

14. A process according to claim 13 wherein from about one to five molar equivalents of a neutralizing agent based on the substrate not present in excess is used with from about three to ten volumes of water based on the weight of the substrate not present in excess.

15. A process according to claim 14 wherein n' = 1 and about three molar equivalents of a neutralizing agent and about five volumes of water are used.

16. A process according to claim 15 wherein the neutralizing agent is sodium carbonate.

17. A process according to claim 16 wherein the piperazine salt, alkyl halide containing compound, sodium carbonate and water are combined and heated to reflux.

18. A process according to claim 17 wherein the temperature of reflux is about 100°C.

19. A process according to claim 13 wherein the compound of formula I is 5-(2-(4-(1,2-benzisothiazol-3-yl)-1-piperazinyl)ethyl)-6-chloro-1,3-dihydro-2H-indol-2-one.

20. A process according to claim 19 comprising the step of reacting the compound of formula I with aqueous hydrochloric add to form 5-(2-(4-(1,2-benzisothiazol-3-yl)-1-piperazinyl)ethyl)-6-chloro-1,3-dihydro-2H-indol-2-one hydrochloride monohydrate.

## Patentansprüche

1. Verfahren zur Herstellung einer Verbindung der Formel 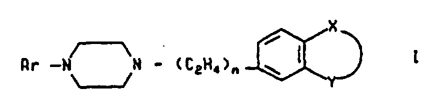 oder eines pharmazeutisch verträglichen Säureadditionssalzes davon, wobei
Ar Naphthyl, gegebenenfalls substituiert mit einem bis vier Substituenten, unabhängig ausgewählt aus Fluor, Chlor, Trifluormethyl, Methoxy, Cyano und Nitro; Chinolyl; 6-Hydroxy-8-chinolyl; Isochinolyl; Chinazolyl; Benzoisothiazolyl und ein Oxid oder Dioxid davon, jeweils gegebenenfalls substituiert mit einem oder mehreren Substituenten, unabhängig ausgewählt aus Fluor, Chlor, Trifluormethyl, Methoxy, Cyano und Nitro; Benzothiazolyl; Benzothiadiazolyl; Benzotriazolyl; Benzoxazolyl; Benzoxazolonyl; Indolyl; Indanyl, gegebenenfalls substituiert mit einem oder zwei Fluoratomen; 3-Indazolyl, gegebenenfalls substituiert mit 1-Trifluormethylphenyl; und Phthalazinyl; darstellt;
n 1 oder 2 ist, und
X und Y, zusammen mit dem Phenylrest, an den sie gebunden sind, ein Ringsystem bilden, ausgewählt aus Chinolyl; 2-Hydroxychinolyl; Benzothiazolyl; 2-Aminobenzothiazolyl; Benzoisothiazolyl; Indazolyl; 2-Hydroxyindazolyl; Indolyl; Spiro[cyclopentan-1,3'-indolinyl]; und Oxindolyl; worin das Ringsystem gegebenenfalls mit einem bis drei Substituenten, unabhängig voneinander ausgewählt aus (C₁-C₃)Alkyl oder mit einem Substituenten, ausgewählt aus Chlor, Fluor, Benzoxazolyl, 2-Aminobenzoxazolyl, Benzoxazolonyl, 2-Aminobenzoxazolinyl, Benzothiazolonyl, Benzoimidazolonyl, Benzotriazolyl und Phenyl, gegebenenfalls substituiert mit einem Chlor- oder Fluoratom, substituiert sein kann;
das Umsetzen eines Piperazins der Formel 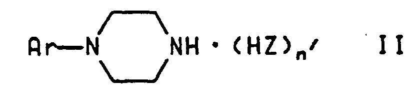 worin Z Fluor, Chlor, Brom, Jod, Methansulfonat, Trifluormethansulfonat oder Trifluoracetat darstellt;
n' 0 oder 1 ist und
Ar wie vorstehend definiert ist, mit einer Alkylhalogenid enthaltenden Verbindung der Formel 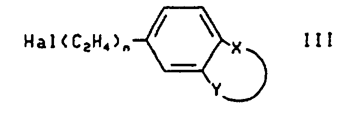
worin n, X und Y wie vorstehend definiert sind, und Hal Fluor, Chlor, Brom oder Jod darstellt, in Wasser mit einem Reagenz zum Neutralisieren der Halogenwasserstoffsäure, Erhitzen des Gemisches unter zum Kuppeln des Piperazins mit der Alkylhalogenid enthaltenden Verbindung geeigneten Bedingungen und, falls erwünscht, Herstellen des entsprechenden pharmazeutisch verträglichen Säureadditionssalzes umfasst.

2. Verfahren nach Anspruch 1, wobei n' = 1 und das Piperazinsalz ein Hydrochlorid- oder Trifluormethansulfonatsalz ist.

3. Verfahren nach Anspruch 1, wobei das Kuppeln des Piperazins mit der Alkylhalogenid enthaltenden Verbindung in Gegenwart eines Neutralisationsmittels bewirkt wird.

4. Verfahren nach Anspruch 3, wobei etwa 1 bis 5 Moläquivalente eines Neutralisationsmittels, bezogen auf das nicht im Überschuss vorliegende Substrat, mit etwa 3 bis 10 Volumen Wasser, bezogen auf das Gewicht des Substrats, das nicht im Überschuss vorliegt, verwendet werden.

5. Verfahren nach Anspruch 4, wobei etwa 3 Moläquivalente Neutralisationsmittel und etwa 5 Volumen Wasser verwendet werden.

6. Verfahren nach Anspruch 5, wobei das Neutralisationsmittel aus der Gruppe, bestehend aus Alkalimetallcarbonaten, Erdalkalimetallcarbonaten, -Bicarbonaten, -Hydriden und tertiären Aminen, ausgewählt ist.

7. Verfahren nach Anspruch 6, wobei das Neutralisationsmittel Natriumcarbonat darstellt.

8. Verfahren nach Anspruch 7, wobei das Piperazin, Alkylhalogenid enthaltende Verbindung, Natriumcarbonat und Wasser vereinigt und unter Rückfluss erhitzt werden.

9. Verfahren nach Anspruch 1, wobei das Gemisch auf etwa die Rückflusstemperatur erhitzt wird.

10. Verfahren nach Anspruch 9, wobei die Rückflusstemperatur etwa 100°C ist.

11. Verfahren nach Anspruch 1, wobei die Verbindung der Formel I 5-(2-(4-(1,2-Benzisothiazol-3-yl)-1-piperazinyl)-ethyl)-6-chlor-1,3-dihydro-2H-indol-2-on darstellt.

12. Verfahren nach Anspruch 11, umfassend den Schritt der Umsetzung der Verbindung der Formel I mit wässriger Salzsäure zu 5-(2-(4-(1,2-Benzisothiazol-3-yl)-1-piperazinyl)-ethyl)-6-chlor-1,3-dihydro-2H-indol-2-onhydrochloridmonohydrat.

13. Verfahren nach Anspruch 1, das Umsetzen des Piperazins mit der Alkylhalogenid enthaltenden Verbindung in Wasser und einem Neutralisationsmittel, Erhitzen des Gemisches für mindestens etwa 8 bis 16 Stunden unter Rückfluss, Abkühlen des Gemisches und Abfiltrieren des Produkts umfasst.

14. Verfahren nach Anspruch 13, wobei etwa 1 bis 5 Moläquivalente eines Neutralisationsmittels, bezogen auf das Substrat, das nicht im Überschuss vorliegt, mit etwa 3 bis 10 Volumen Wasser, bezogen auf das Gewicht des Substrats, das nicht im Überschuss vorliegt, verwendet werden.

15. Verfahren nach Anspruch 14, wobei n' = 1 und etwa 3 Moläquivalente eines Neutralisationsmittels und etwa 5 Volumen Wasser verwendet werden.

16. Verfahren nach Anspruch 15, wobei das Neutralisationsmittel Natriumcarbonat ist.

17. Verfahren nach Anspruch 16, wobei das Piperazinsalz, Alkylhalogenid enthaltende Verbindung, Natriumcarbonat und Wasser vereinigt und unter Rückfluss erhitzt werden.

18. Verfahren nach Anspruch 17, wobei die Rückflusstemperatur etwa 100°C ist.

19. Verfahren nach Anspruch 13, wobei die Verbindung der Formel I 5-(2-(4-(1,2-Benzisothiazol-3-yl)-1-piperazinyl)-ethyl)-6-chlor-1,3-dihydro-2H-indol-2-on ist.

20. Verfahren nach Anspruch 19, umfassend den Schritt der Umsetzung der Verbindung der Formel I mit wässriger Salzsäure zu 5-(2-(4-(1,2-Benzisothiazol-3-yl)-1-piperazinyl)-ethyl)-6-chlor-1,3-dihydro-2H-indol-2-onhydrochloridmonohydrat.

## Revendications

1. Procédé pour la préparation d'un composé de formule : 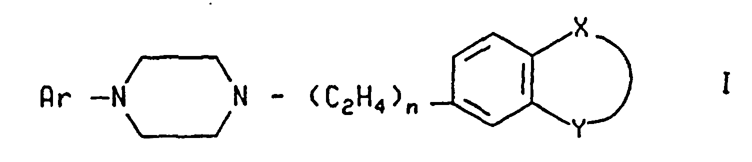 ou d'un de ses sels d'addition d'acides pharmaceutiquement acceptables, formule dans laquelle
Ar représente un groupe naphtyle facultativement substitué avec un à quatre substituants choisis indépendamment entre des substituants fluoro, chloro, trifluorométhyle, méthoxy, cyano et nitro ; quinolyle ; 6-hydroxy-8-quinolyle ; isoquinolyle ; quinazolyle ; benzisothiazolyle ou un de ses oxydes ou dioxydes, chacun étant facultativement substitué avec un ou plusieurs substituants choisis indépendamment entre des substituants fluoro, chloro, trifluorométhyle, méthoxy, cyano et nitro ; benzothiazolyle ; benzothiadiazolyle ; benzotriazolyle ; benzoxazolyle ; benzoxazolonyle ; indolyle ; indanyle facultativement substitué avec un ou deux substituants fluoro ; 3-indazolyle facultativement substitué avec un substituant 1-trifluorométhylphényle ; ou phtalazinyle ;
n est égal à 1 ou 2 ; et
X et Y, conjointement avec le groupe phényle auquel ils sont fixés, forment un système cyclique choisi entre quinolyle ; 2-hydroxyquinolyle ; benzothiazolyle ; 2-aminobenzothiazolyle ; benzisothiazolyle ; indazolyle ; 2-hydroxyindazolyle ; indolyle ; spiro[cyclopentane-1,3'-indolinyle] ; et oxindolyle ; ledit système cyclique pouvant être facultativement substitué avec un à trois substituants choisis indépendamment parmi des substituants alkyle en C₁ à C₃ ; ou avec un substituant choisi entre des substituants chloro, fluoro, benzoxazolyle, 2-aminobenzoxazolyle, benzoxazolonyle, 2-aminobenzoxazolinyle, benzothiazolonyle, benzimidazolonyle, benzotriazolyle et phényle facultativement substitué avec un substituant chloro ou fluoro ;
qui comprend la réaction d'une pipérazine de formule 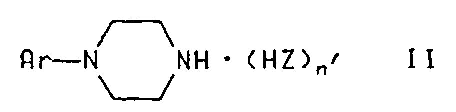 dans laquelle Z représente un groupe fluoro, chloro, bromo, iodo, méthanesulfonate, trifluorométhanesulfonate ou trifluoracétate ;
n' est égal à 0 ou 1 ; et
Ar répond à la définition précitée, avec un composé à fonction halogénure d'alkyle de formule 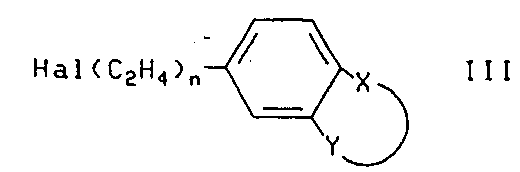 dans laquelle n, X et Y répondent aux définitions précitées et Hal représente un groupe fluoro, chloro, bromo ou iodo, dans de l'eau avec un réactif pour neutraliser l'acide halogénhydrique, le chauffage du mélange dans des conditions qui conviennent pour effectuer le couplage de ladite pipérazine avec ledit composé à fonction halogénure d'alkyle et, si cela est désiré, la préparation du sel d'addition d'acide pharmaceutiquement acceptable correspondant.

2. Procédé suivant la revendication 1, dans lequel n' est égal à 1 et le sel de pipérazine est un chlorhydrate ou triflate.

3. Procédé suivant la revendication 1, dans lequel le couplage de la pipérazine avec le composé à fonction halogénure d'alkyle est effectué en présence d'un agent neutralisant.

4. Procédé suivant la revendication 3, dans lequel une quantité d'environ un à cinq équivalents molaires d'un agent neutralisant sur la base du substrat non présent en excès est utilisée avec une quantité d'environ trois à dix volumes d'eau sur la base du poids du substrat non présent en excès.

5. Procédé suivant la revendication 4, dans lequel environ trois équivalents molaires d'un agent neutralisant et environ cinq volumes d'eau sont utilisés.

6. Procédé suivant la revendication. 5, dans lequel l'agent neutralisant est choisi dans le groupe consistant en des carbonates de métaux alcalins, des carbonates de métaux alcaline-terreux, des bicarbonates, des hydrures et des amines tertiaires.

7. Procédé suivant la revendication 6, dans lequel l'agent neutralisant est le carbonate de sodium.

8. Procédé suivant la revendication 7, dans lequel la pipérazine, le composé à fonction halogénure d'alkyle, le carbonate de sodium et l'eau sont mélangés et chauffés au reflux.

9. Procédé suivant la revendication 1, dans lequel le mélange est chauffé à une température approximativement égale à la température du reflux.

10. Procédé suivant la revendication 9, dans lequel la température de reflux est égale à environ 100°C.

11. Procédé suivant la revendication 1, dans lequel le composé de formule I est la 5-(2-(4-(1,2-benzisothiazole-3-yl)-1-pipérazinyl)éthyl)-6-chloro-1,3-dihydro-2H-indole-2-one.

12. Procédé suivant la revendication 11, comprenant l'étape consistant à faire réagir le composé de formule I avec une solution aqueuse d'acide chlorhydrique pour former le monohydrate de chlorhydrate de 5-(2-(4-(1,2-benzisothiazole-3-yl)-1-pipérazinyl)éthyl)-6-chloro-1,3-dihydro-2H-indole-2-one.

13. Procédé suivant la revendication 1, qui comprend la réaction de la pipérazine avec le composé à fonction halogénure d'alkyle dans de l'eau et un agent neutralisant ; le chauffage du mélange au reflux pendant un temps d'au moins environ 8 à 16 heures ; le refroidissement du mélange et la séparation du produit par filtration.

14. Procédé suivant la revendication 13, dans lequel une quantité d'environ un à cinq équivalents molaires d'un agent neutralisant sur la base du substrat non présent en excès est utilisée avec une quantité d'environ trois à dix volumes d'eau sur la base du poids du substrat non présent en excès.

15. Procédé suivant la revendication 14, dans lequel n' est égal à 1 et environ trois équivalents molaires d'un agent neutralisant et environ cinq volumes d'eau sont utilisés.

16. Procédé suivant la revendication 15, dans lequel l'agent neutralisant est le carbonate de sodium.

17. Procédé suivant la revendication 16, dans lequel le sel de pipérazine, le composé à fonction halogénure d'alkyle, le carbonate de sodium et l'eau sont mélangés et chauffés au reflux.

18. Procédé suivant la revendication 17, dans lequel la température de reflux est égale à environ 100°C.

19. Procédé suivant la revendication 13, dans lequel le composé de formule I est la 5-(2-(4-(1,2-benzisothiazole-3-yl)-1-pipérazinyl)éthyl)-6-chloro-1,3-dihydro-2H-indole-2-one.

20. Procédé suivant la revendication 19, comprenant l'étape consistant à faire réagir le composé de formule I avec une solution aqueuse d'acide chlorhydrique pour former le monohydrate de chlorhydrate de 5-(2-(4-(1,2-benzisothiazole-3-yl)-1-pipérazinyl)éthyl)-6-chloro-1,3-dihydro-2H-indole-2-one.
